**Europäisches Patentamt**

**European Patent Office**

**Office européen des brevets**

(19)

(11) Veröffentlichungsnummer: **0 289 512 B1**

# (12) EUROPÄISCHE PATENTSCHRIFT

(45) Veröffentlichungstag der Patentschrift :
**14.08.91 Patentblatt 91/33**

(51) Int. Cl.$^5$ : **A61K 47/00, A61K 31/725**

(21) Anmeldenummer : **87900720.1**

(22) Anmeldetag : **15.12.86**

(86) Internationale Anmeldenummer :
**PCT/EP86/00750**

(87) Internationale Veröffentlichungsnummer :
**WO 87/04350 30.07.87 Gazette 87/17**

(54) **VERFAHREN ZUR VERBESSERUNG DER HAFTFÄHIGKEIT VON GELEN AUF DER SCHLEIMHAUT.**

(30) Priorität : **16.01.86 DE 3601132**

(43) Veröffentlichungstag der Anmeldung :
**09.11.88 Patentblatt 88/45**

(45) Bekanntmachung des Hinweises auf die
Patenterteilung :
**14.08.91 Patentblatt 91/33**

(84) Benannte Vertragsstaaten :
**AT BE CH DE FR GB IT LI LU NL SE**

(56) Entgegenhaltungen :
**EP-A- 0 048 123**
**EP-A- 0 059 221**
**BE-A- 670 837**
**DE-A- 2 224 700**

(73) Patentinhaber : **BANNERT, Christian**
**Miltenbergstrasse 17**
**W-8900 Augsburg 22 (DE)**

(72) Erfinder : **BANNERT, Christian**
**Miltenbergstrasse 17**
**W-8900 Augsburg 22 (DE)**

(74) Vertreter : **Weickmann, Heinrich, Dipl.-Ing. et**
**al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**W-8000 München 86 (DE)**

## Beschreibung

Der Erfindung betrifft ein untenstehend näher definiertes Mittel mit guter Haftung auf der Schleimhaut.

Zur Behandlung von Erkrankungen der Schleimhäute hat es sich als nützlich erwiesen, Gele zu verwenden. Zum einen können sie eingesetzt werden zum Schutz und zum Feuchthalten von Schleimhäuten. Andererseits werden sie verwendet, um Desinfizientia und Arzneistoffe auf die Schleimhaut aufzubringen und dort wirken zu lassen. Diese Gele, die aus synthetischen oder pflanzlichen Stoffen hergestellt werden und verschiedene Wirkstoffe enthalten können, werden auf die Schleimhaut im Nasen-, Mund-, Rachen-, Urogenitalbereich und Gastrointestinaltrakt aufgetragen.

So ist es beispielsweise aus DE-A-3152319 sowie aus EP-A-0059221 bekannt, für Blutungen im Gastrointestinaltrakt wasserlösliche Alginate in Form eines Geles zur Blutstillung zu verwenden. Nachteil dieser bekannten Substanzen ist es jedoch, daß sie auf der Schleimhaut schlecht haften, so daß sie an der Stelle, an der sie aufgetragen werden und wirken sollen, nicht allzu lange verweilen. Sie perlen von der Schleimhaut ab und können die erwünschte Wirkung nicht erzielen.

Weiterhin ist es aus DE-A-2224700 bekannt, einen Film herzustellen aus einem wasserunlöslichen Metallsalz eines filmbildenden Materials, wie beispielsweise Alginsäure. Aus dem so hergestellten Film können dann Scheiben oder kleine Stücke ausgeschnitten und dann auf Wunden aufgelegt werden. Auch hier ist der Nachteil, daß die aufgelegten Filmstücke verrutschen, da sie auf der Schleimhaut nicht haften.

Aus BE-B-670837 ist es weiterhin bekannt, für therapeutische und kosmetische Zwecke auf die Haut einen Film aus einem wasserunlöslichen Salz der Alginsäure aufzubringen. Hier wird auf die Haut zuerst eine Lösung von Alginsäure mit einer ausreichenden Viskosität, um auf der Haut zu haften, aufgebracht. Anschließend wird eine Calciumsalzlösung auf diesen Film aufgebracht, um das unlösliche Calciumalginat auf diese Weise zu koagulieren. Auf diese Weise wird ein rein mechanisch wirkender Schutzverband auf der Haut aufgebracht. Auch auf diese Weise ist es nicht möglich, einen gut haftenden Film auf die Schleimhaut aufzubringen.

Aufgabe der vorliegenden Erfindung war es nun, diese bekannten Nachteile zu verhindern und ein Mittel zur Verfügung zu stellen, bei dem die Haftfähigkeit auf der Schleimhaut verbessert ist.

Dieses Ziel wird erreicht durch ein Mittel mit guter Haftung auf der Schleimhaut, das dadurch gekennzeichnet ist, daß es in situ erhalten wird, indem zuerst eine ein Metallsalz, das zur Gelbildung mit einem Polysaccharid befähigt ist, enthaltende Lösung auf die Schleimbaut aufgebracht und anschließend eine ein zur Gelbildung befähigtes Polysaccharid enthaltende Lösung auf denselben Bereich der Schleimhaut aufgebracht wird.

Es hat sich überraschenderweise gezeigt, daß dann, wenn nicht das fertige Gel, sondern die miteinander zur Gelbildung befähigten Komponenten — zuerst die Metallsalzlösung und dann die Polysaccharidlösung — auf die Schleimhaut aufgetragen werden, die dann auf der Schleimhaut ein Gel bilden, eine gute Haftung erreicht werden kann.

Die beiden zur Gelbildung befähigten Komponenten werden getrennt voneinander gelöst. Die Lösungen A, und B, werden dann nacheinander aufgetragen. Das Auftragen kann in üblicher Weise geschehen. Bevorzugt werden die beiden Komponenten auf die Schleimhaut aufgesprüht, durch Trinken der beiden Lösungen oder durch Sonden bzw. Katheter auf die entsprechende Stelle aufgebracht.

Als zur Gelbildung befähigte Komponenten können Metallsalze und Polysaccharide verwendet werden, die bei der Vermischung zu einer Gelbildung führen. Sie müssen darüberhinaus schleimhautverträglich sein und dürfen nicht toxisch sein. Geeignet sind beispielsweise Kombinationen von Alginsäure, Polyguluronsäure, Polymannuronsäure, Propylenglykolalginsäure, Polygalacturonsäure, deren Salze oder Ester oder deren Mischungen sowie Pectin mit, pharmazeutisch verträglichen Metallsalzen.

Bevorzugt werden als gelbildende Komponenten Calciumsalze zusammen mit Alginsäure oder einem ihrer Derivate oder mit einem Pektin mit niedrigem Veresterungsgrad eingesetzt. Calciumsalze reagieren mit Alginsäure und Pektinen und deren Derivaten und bilden Quervernetzungen. Mit steigendem Gehalt des Metallsalzes tritt zunächst Verdickung und dann Gelbildung ein. Als Metallsalz werden bevorzugt Calciumsalze verwendet, wobei als Calciumsalze alle pharmazeutisch verträglichen Salze verwendet werden können wie Calciumchlorid oder Calciumsalze mit organischen Anionen wie Citrat, Lactat, Aspartat, Saccharat, Oxovalerat, Gluconat, Lactobionat und Lactogluconat. Bevorzugt wird Calciumgluconat verwendet.

Als Alginsäurederivat wird bevorzugt Natriumalginat, von dem alle Viskositätstypen zur Anwendung kommen können. Sie werden durch die Viskosität der 1%igen Lösung klassifiziert. Die Messung erfolgt bei 25°C mit einem Brookfield-Viskosimeter. Handelsübliche Alginattypen sind :

sehr niedrigviskoses Natriumalginat (5 cps),
niedrigviskoses Natriumalginat (50 cps),
hochviskoses Natriumalginat (400 cps),

sehr hochviskoses Natriumalginat (1350 cps).

Neben der Alginsäure sind in den Braunalgen noch andere Mono- und Polysaccharide enthalten. Bedeutsam für die Schleimhäute sind dabei die Fucose und die Estersulfate. Durch geeignete Methoden zur Aufarbeitung der Braunalgen können Gemische aus Alginaten, Fucose und Estersulfaten gewonnen werden. Diese Gemische eignen sich ebenfalls als Komponenten zur Gelbildung in dem erfindungsgemäßen Verfahren.

Als Pektin wird bevorzugt ein Pektin mit niedrigem Veresterungsgrad verwendet. Beispielsweise geeignet sind Polygalacturonsäuren mit einem Anteil von etwa 37 bis 39% methoxylierten Carboxylgruppen.

Die beiden Komponenten werden gelöst verwendet. Dabei wird bevorzugt das Metallsalz in einer Konzentration von 0,1 bis 50 mMol Metall/100 ml und das Polysaccharid in einer Konzentration von 0,1 bis 12,5 Gew.-% verwendet. Die jeweilig einzusetzende Konzentration kann leicht bestimmt werden. Sie ist auch abhängig von dem Viskositätsgrad des verwendeten Polysaccharids.

Als Lösungsmittel werden pharmazeutisch verträgliche Mittel verwendet, die die jeweilige Komponente gut lösen. Bevorzugt wird destilliertes Wasser verwendet, das mit einem pharmazeutisch verträglichen Konservierungsmittel versehen ist.

Das erfindungsgemäß gebildete Gel kann dazu verwendet werden, die Schleimhaut feucht zu halten oder zu schützen. Weiterhin kann in einer weiteren Ausführungsform mit einer der erfindungsgemäß verwendeten Komponenten noch zusätzlich ein Desinfektionsmittel und/oder Arzneimittel auf die Schleimhaut aufgebracht werden. Das erfindungsgemäß auf der Schleimhaut entstehende Gel dient dann zur Behandlung von Krankheiten, die die Schleimhaut betreffen.

Die erfindungsgemäß erhaltenen Gele werden bevorzugt als Trägermaterial für ein Arzneimittel verwendet. Dazu wird dann in eine der beiden Komponentenlösungen das Arzneimittel in der zu applizierenden Dosis eingebracht und dann die beiden Komponenten auf die Schleimhaut aufgetragen.

Ein weiterer Gegenstand der Erfindung ist ein Mittel mit guter Haftung auf der Schleimhaut, das dadurch gekennzeichnet ist, daß es als zur Gelbildung miteinander befähigte Komponenten als Metallsalz ein organisches Calciumsalz und als Polysaccharid Alginsäure, Polyguluronsaure, Polymannuronsäure, Propylenglykolalginsäure, Polygalacturonsäure, deren Salze oder Ester, Pektin oder deren Mischungen physikalisch getrennt voneinander enthält.

Ein weiterer Gegenstand der Erfindung ist ein Mittel mit guter Haftung auf der Schleimhaut, das dadurch gekennzeichnet ist, daß es mindestens zwei miteinander zur Gelbildung befähigte Komponenten physikalisch getrennt voneinander enthält.

Mit dem erfindungsgemäßen Mittel können Gele in einfacher Weise auf der Schleimhaut gebildet werden, die lange und dauerhaft auf der Schleimhaut haften.

Als Komponenten des erfindungsgemäßen Mittels, die physikalisch getrennt voneinander vorliegen, werden bevorzugt mindestens ein Metallsalz und mindestens ein Polysaccharid verwendet. Als Metallsalz wird bevorzugt ein Calciumsalz eingesetzt und besonders bevorzugt wird Calciumchlorid oder ein organisches Calciumsalz verwendet. Als Polysaccharid wird besonders bevorzugt Alginsäure, Polyguluronsäure, Polymannuronsäure, Propylenglykolalginsäure, Polygalacturonsäure, deren Salze oder Ester sowie Pektin, insbesondere mit einem niedrigen Veresterungsgrad und deren Mischungen verwendet.

Die beiden Komponenten sind bevorzugt in Wasser gelöst. Dabei enthält die eine Lösung bevorzugt 0,1 bis 50 mMol Metall/100 ml, während die andere Lösung bevorzugt 0,1 bis 12,5 Gew.-% mindestens eines Polysaccharids enthält.

In einer bevorzugten Ausführungsform enthält mindestens eine der beiden Komponenten des erfindungsgemäßen Mittels zusätzlich noch Arzneistoffe, Desinfizienzia, Feuchthaltemittel und/oder Konservierungsmittel oder sonst übliche Zusatzstoffe. So wird beispielsweise zur Erhöhung der Flexibilität des Gels dem Mittel Sorbit oder Glycerin zugegeben. Weiterhin kann es geeignet sein, Tenside zuzusetzen, um die Benetzung zu verbessern.

Die Konzentration der beiden Komponenten des erfindungsgemäßen Mittels ist in der Regel jeweils in etwa gleich. In speziellen Fällen kann es jedoch vorteilhaft sein, eine der beiden Lösungen mit höherer Konzentration und dafür geringerem Volumen einzusetzen. Durch die Konzentration kann auch die Art des entstehenden Gels beeinflußt werden. So bilden beispielsweise Lösungen niedriger Konzentrationen von Alginat bzw. Pektin einerseits und Metallsalz andererseits dünne Gelfilme während beim Einsatz der Komponenten in hoher Konzentration feste Gelfilme erhalten werden.

Dünne Gelfilme eignen sich insbesondere als Speichelersatz im Mund- und Rachenbereich und als künstliches Nasensekret in der Nase. Diese dünnen Gelfilme können z.B. erhalten werden, wenn man gleiche Volumina einer Calciumsalzlösung und einer Natriumalginatlösung verwendet, wobei Konzentrationen von 0,1 bis 7 mMol Calcium/100 ml bzw, 0,1 bis 5 Gew.-M Natriumalginat verwendet werden. Bevorzugt wird hier zuerst die Calciumsalzlösung aufgesprüht und anschließend die Alginatlösung aufgesprüht. Für die Schleimhaut des Ösophagus, die durch Sprühen nicht erreicht werden kann, eignet sich am besten das Aufbringen durch Trinken der beiden Komponentenlösungen. Für den Gastrointestinalbereich erfolgt das Auftragen über Sonden oder Katheter. Im Urogenitalbereich werden die Gelfilme durch Aufsprühen oder Auftragen über Sonden oder Katheter erzeugt.

Verwendet man Lösungen mit höheren Konzentrationen, so entstehen festere Gelschichten. Für diesen Fall hat es sich als bevorzugt erwiesen, zuerst die Metallsalzkomponente aufzubringen und anschließend die Polysaccharidkomponente. Von den beiden gelöst vorliegenden Komponenten hat insbesondere bei höheren Konzentrationen, die Metallsalzlösung eine geringere Viskosität. Diese niedrigviskose Lösung kann in die Falten und Unebenheiten der Schleimhaut eindringen. Gibt man dann die höherviskose Polysaccharidlösung zu, so tritt die Gelbildung auch in den Falten und Unebenheiten der Schleimhaut auf und die Haftung ist aus diesem Grunde sehr gut. Diese festen Gelfilme decken die geschädigte Schleimhaut ab und schützen sie vor Magensäure, Enzymen, Bakterien oder anderen schädlichen Einflüssen von außen. Mit Hilfe des erfindungsgemäßen Verfahrens kann der feste Gelfilm an der gewünschten Stelle gezielt aufgebracht werden, indem man z.B. über eine Sonde oder einen Katheter die beiden Komponenten auf das gewünschte Gebiet aufträgt, auftropft oder gießt. Diese Art der Applikation eignet sich z.B. für Erkrankungen der Schleimhaut, die durch Reflux Ösophagitis, Sklerotisierungsulcera im Ösophagus, Magenulcera, Morbus Crohn und Strahlenulcera im Dickdarm hervorgerufen werden.

Die erfindungsgemäß erhaltenen, besonders haftfähigen Gele sind insbesondere geeignet zur Verwendung als Trägermaterial für Arzneimittel oder Desinfizienzia. Unter Verwendung des mit dem erfindungsgemäßen Verfahren erhaltenen Gels lassen sich Arzneimittel und Desinfizienzia gezielt lokal applizieren.

Die folgenden Beispiele erläutern die Erfindung : Alle Prozentangaben beziehen sich auf das Gewicht, sofern nichts anderes angegeben ist.

## Beispiel 1

Es wurde ein Mittel zum Schutz der Schleimhaut hergestellt. Dazu wurde eine Lösung, die Calciumgluconat in einer Konzentration von 0,5% in destilliertem Wasser enthielt, hergestellt. Diese Lösung wurde mit einem Konservierungsmittel versetzt. Weiterhin wurde eine Lösung hergestellt, die 0,5% Natriumalginat in destilliertem Wasser enthielt. Diese Lösung wurde ebenfalls mit einem Konservierungsmittel versetzt. Die beiden Lösungen wurden auf die Schleimhaut aufgesprüht. Es bildete sich ein Gel, das lange auf der Schleimhaut haftete.

## Beispiel 2

Es wurde ein Mittel zur Desinfektion von Schleimhaut hergestellt. Dazu wurden 0,5 Gew.-Teile Calciumgluconat in destilliertem Wasser gelöst und mit Konservierungsmittel versetzt. Weiterhin wurden 0,5 Gew.-Teile Natriumalginat und 0,2 Gew.-Teile 4-Chlor-3-Methylphenol in destilliertem Wasser gelöst. Beide Lösungen wurden gleichzeitig auf die Schleimhaut aufgesprüht. Es bildete sich ein Gel, das auf der Schleimhaut haftete.

## Beispiel 3

Es wurde ein Mittel hergestellt, das als künstlicher Speichel im Rachenraum verwendet werden kann. Die beiden Komponenten hatten die folgende Zusammensetzung :

| Lösung A | | Lösung B | |
|---|---|---|---|
| Calciumgluconat | 0,5 % | Na-Alginat, hochviskos | 0,75 % |
| Kochsalz | 0,1 % | Kochsalz | 0,1 % |
| Kaliumchlorid | 0,2 % | Kaliumchlorid | 0,2 % |
| Sorbit | 0,2 % | Sorbit | 0,2 % |
| Wasser | ad 100 ml | Wasser | ad 100 ml |

## Beispiel 4

Es wurde ein Mittel, das als künstlicher Speichel dienen kann, hergestellt mit der folgenden Zusammensetzung :

4

```
Lösung A                          Lösung B
Calciumgluconat..     1 %   Na-Alginat, niedrigviskos    1 %
Wasser           ad 100 ml  Pektin                       0,2 %
                            Wasser                  ad 100 ml
```

**Beispiel 5**

Es wurde ein Mittel wie in Beispiel 3 hergestellt, dem zur Verbesserung der Benetzung ein Tensid zugesetzt wurde. Das Mittel hatte die folgende Zusammensetzung :

```
Lösung A                                Lösung B
Calciumgluconat        0,5%     Na-Alginat, niedrigviskos  0,75 %
Cremophor® El (Tensid) 0,025 %  Wasser               ad 100 ml
Sorbit                 1 %
Wasser            ad 100 ml
```

**Beispiel 6**

Es wurde ein als künstlicher Speichel geeignetes Mittel hergestellt mit der folgenden Zusammensetzung:

```
Lösung A                          Lösung B
Calciumgluconat       3 %   Na-Alginat, sehr hochviskos  0,1 %
Sorbit                5 %   Sorbit                       5 %
Wasser           ad 100 ml  Wasser                  ad 100 ml
```

**Beispiel 7**

Es wurde ein Mittel hergestellt, das als Trägermittel für Neuraminsäure als Wirkstoff dient :

```
Lösung A                    Lösung B
Calciumgluconat      0,1%   Natrium-Alginat, sehr niedrigviskos  5 %
Wasser          ad 100 ml   Neuraminsäure                        0,1 %
                            Wasser                          ad 100 ml
```

**Beispiel 8**

Es wurde ein Mittel hergestellt, das als künstliches Nasensekret verwendet werden kann und direkt in die Nase eingesprüht wird :

```
Lösung A                        Lösung B
Calciumgluconat   0,5 %   Natrium-Alginat, niedrigviskos  1,5 %
Natriumchlorid    0,75 %  Natriumchlorid                  0,75 %
Kaliumchlorid     0,13 %  Kaliumchlorid                   0,13 %
Sorbit            0,5 %   Sorbit                          0,5 %
Wasser       ad 100 ml    Wasser                     ad 100 ml
```

**Beispiel 9**

Zum Feuchthalten der Nasenschleimhaut wurde das folgende Mittel hergestellt :

| Lösung A | | Lösung B | |
|---|---|---|---|
| Calciumgluconat | 0,75 % | Natrium-Alginat, niedrigviskos | 2 % |
| Natriumchlorid | 1,5 % | Natriumchlorid | 1,5 % |
| Kaliumchlorid | 0,23 % | Kaliumchlorid | 0,23 % |
| Sorbit | 0,5 % | Sorbit | 0,5 % |
| Wasser | ad 100 ml | Wasser | ad 100 ml |

**Beispiel 10**

Es wurde ein bettel zur Verwendung als künstliches Nasensekret hergestellt :

| Lösung A | | Lösung B | |
|---|---|---|---|
| Calciumgluconat | 2 % | Natrium-Alginat, niedrigviskos | 1 % |
| Sorbit | 5 % | | |
| Wasser | ad 100 ml | Wasser | ad 100 ml |

**Beispiel 11**

Es wurde ein Mittel hergestellt, das zum Aufbringen von Xylometazolin als Vasoconstringens auf die Nasenschleimhaut verwendet wurde :

| Lösung A | | Lösung B | |
|---|---|---|---|
| Calciumgluconat | 0,5 % | Natrium-Alginat, niedrigviskos | 1,5 % |
| Natriumchlorid | 0,75 % | Natriumchlorid | 0,75 % |
| Kaliumchlorid | 0,13 % | Kaliumchlorid | 0,13 % |
| Sorbit | 0,5 % | Sorbit | 0,5 % |
| Xylometazolin | 0,1 % | Wasser | ad 100 ml |
| Wasser | ad 100 ml | | |

**Beispiel 12**

Es wurde ein Mittel hergestellt, das zum Schutz der Schleimhaut dient :

| Lösung A | | Lösung B | |
|---|---|---|---|
| Calciumgluconat | 1 % | Pektin | 1 % |
| Wasser | ad 100 ml | Wasser | ad 100 ml |

**Beispiel 13**

Es wurde ein Mittel hergestellt, das zum Aufbringen von Neuraminsäure auf die Nasenschleimhaut verwendet wurde :

| Lösung A | | Lösung B | |
|---|---|---|---|
| Calciumgluconat | 0,1 % | Natrium-Alginat, sehr niedrigviskos | 5 % |
| Wasser | ad 100 ml | Neuraminsäure | 0,1 % |
| | | Wasser | ad 100 ml |

**Beispiel 14**

Es wurde ein Mittel hergestellt, das auf die Schleimhaut des Ösophagus durch Trinken aufgebracht wird :

| Lösung A | | Lösung B | |
|---|---|---|---|
| Calciumgluconat | 2 % | Natrium-Alginat, niedrigviskos | 1,5 % |
| Glycerin | 2,5 % | Sorbit | 1 % |
| Wasser | ad 100 ml | Wasser | ad 100 ml |

**Beispiel 15**

Es wurde ein Mittel hergestellt, das gut auf der Schleimhaut des Osophagus haftet :

| Lösung A | | Lösung B | |
|---|---|---|---|
| Calciumgluconat | 10 % | Natrium-Alginat, niedrigviskos | 1,5 % |
| Calciumlactobionat | 17,5 % | Sorbit | 1 % |
| Wasser | ad 100 ml | Wasser | ad 100 ml |

**Beispiel 16**

Es wurde ein Mittel zum Aufbringen auf die Schleimhaut des Ösophagus hergestellt :

| Lösung A | | Lösung B | |
|---|---|---|---|
| Calciumlactogluconat | 5 % | Natrium-Alginat, sehr niedrigviskos | 5 % |
| Calciumcarbonat | 0,9 % | Wasser | ad 100 ml |
| Zitronensäure | 3,4 % | | |
| Wasser | ad 100 ml | | |

**Beispiel 17**

Es wurde ein Mittel zum Aufbringen auf die Schleimhaut des Ösophagus hergestellt :

| Lösung A | | Lösung B | |
|---|---|---|---|
| Calciumgluconat | 10 % | Natrium-Alginat, sehr niedrigviskos | 12,5 % |
| Calciumlactobionat | 17,5 % | Wasser | ad 100 ml |
| Wasser | ad 100 ml | | |

**Beispiel 18**

Es wurde ein Mittel hergestellt, das zum Auftragen auf Magen Ulcera verwendet wurde :

| Lösung A | | Lösung B | |
|---|---|---|---|
| Calciumgluconat | 2 % | Natrium-Alginat, niedrigviskos | 1,5 % |
| Tensid | 1 % | Sorbit | 1 % |
| Glycerin | 2,5 % | Wasser | ad 100 ml |
| Wasser | ad 100 ml | | |

**Beispiel 19**

Es wurde ein Mittel hergestellt, das als Trägermittel für Misoprostol als Prostaglandin zur Behandlung eines Magenulcus verwendet wurde :

| Lösung A | | Lösung B | |
|---|---|---|---|
| Calciumgluconat | 10 % | Natrium-Alginat, niedrigviskos | 1,5 % |
| Calciumlactobionat | 8,75 % | Misoprostol | 0,4 mg |
| Wasser | ad 100 ml | Wasser | ad 100 ml |

**Beispiel 20**

Es wurde ein Mittel hergestellt, das als Trägermittel für Sucralfat zur Behandlung von Magenulcera diente:

| Lösung A | | Lösung B | |
|---|---|---|---|
| Calciumgluconat | 10 % | Natrium-Alginat, sehr niedrigviskos | 5 % |
| Calciumlactobionat | 17,5 % | Sucralfat | 1 % |
| Wasser | ad 100 ml | Wasser | ad 100 ml |

**Beispiel 21**

Es wurde ein Mittel hergestellt, das als Trägermittel für 5-Aminosalicylsäure zur Behandlung von Colitis Ulcerosa verwendet wurde :

| Lösung A | | Lösung B | |
|---|---|---|---|
| Calciumgluconat | 2 % | 5-Aminosalicylsäure | 4 % |
| Tensid | 1 % | $NaH_2PO_4 x H_2O$ | 0,475 % |
| Glycerin | 2,5 % | $Na_2HPO_4 x 7H_2O$ | 0,075 % |
| Wasser | ad 100 ml | Natriumchlorid | 0,9 % |
| | | Natriumascorbat | 0,05 % |
| | | Natrium-Alginat, niedrigviskos | 1 % |
| | | Propylenglykol | 2,5 % |
| | | Wasser | ad 100 ml |

**Beispiel 22**

Es wurde ein Mittel hergestellt, das als Trägermittel für 5-Aminosalicylsäure zur Behandlung von Colitis Ulcerosa verwendet wurde :

| Lösung A | | Lösung B | |
|---|---|---|---|
| Calciumgluconat | 10 % | 5-Aminosalicylsäure | 4 % |
| Calciumlactobionat | 8,75 % | $NaH_2PO_4xH_2O$ | 0,475 % |
| Wasser | ad 100 ml | $Na_2HPO_4x7H_2O$ | 0,075 % |
| | | Natriumchlorid | 0,9 % |
| | | Natriumascorbat | 0,05 % |
| | | Natriumalginat, | |
| | | sehr niedrigviskos | 5 % |
| | | Propylenglykol | 2,5 % |
| | | Wasser | ad 100 ml |

## Beispiel 23

Es wurde ein Mittel hergestellt, das zum Aufbringen auf die Schleimhaut im Rectum verwendet wurde und für die Behandlung von Strahlencolitis geeignet ist :

| Lösung A | | Lösung B | |
|---|---|---|---|
| Calciumgluconat | 2 % | Natrium-Alginat, niedrigviskos | 1 % |
| Tensid | 1 % | Sorbit | 1 % |
| Glycerin | 2,5 % | Wasser | ad 100 ml |
| Wasser | ad 100 ml | | |

## Beispiel 24

Es wurde ein Mittel hergestellt, das zum Aufbringen auf die Schleimhaut im Rectum verwendet werden kann :

| Lösung A | | Lösung B | |
|---|---|---|---|
| Calciumgluconat | 10 % | Natrium-Alginat, sehr niedrigviskos | 5 % |
| Calciumlactobionat | 8,75 % | Wasser | ad 100 ml |
| Wasser | ad 100 ml | | |

## Beispiel 25

Es wurde ein Mittel hergestellt, das auf die Schleimhaut des Rectum aufgebracht werden kann :

| Lösung A | | Lösung B | |
|---|---|---|---|
| Calciumgluconat | 10 % | Pektin | 2 % |
| Calciumlactobionat | 8,75 % | Wasser | ad 100 ml |
| Wasser | ad 100 ml | | |

## Beispiel 26

Es wurde ein Mittel hergestellt, das geeignet ist zum Aufbringen auf die Schleimhaut der Vagina, insbesondere zur Behandlung von Ulcerationen :

| Lösung A | | | Lösung B | |
|---|---|---|---|---|
| Calciumchlorid x $2H_2O$ | 6,6 % | | Pektin | 5 % |
| Wasser | | ad 100 ml | Wasser | ad 100 ml |

**Beispiel 27**

Es wurde ein Mittel hergestellt, das zur Feuchthaltung der Vaginalschleimhaut verwendet werden kann :

| Lösung A | | Lösung B | |
|---|---|---|---|
| Calciumgluconat | 2 % | Natrium-Alginat, niedrigviskos | 1,5 % |
| Glycerin | 2,5 % | Sorbit | 1 % |
| Wasser | ad 100 ml | Wasser | ad 100 ml |

**Beispiel 28**

Es wurde ein Mittel hergestellt, das als Träger für Metronidazol zur lokalen Behandlung von Infektionen im Vaginalbereich verwendet wird :

| Lösung A | | Lösung B | |
|---|---|---|---|
| Calciumgluconat | 10 % | Natrium-Alginat, sehr niedrigviskos | 5 % |
| Calciumlactobionat | 8,75 % | Metronidazol | 0,5 % |
| Wasser | ad 100 ml | Wasser | ad 100 ml |

**Beispiel 29**

Ein Patient, 62 Jahre, litt nach einer Hartstrahlenbehandlung unter Xerostomie. Der Mund und Rachenraum wurde mit den Lösungen A und B von Beispiel 3 besprüht. Es bildete sich ein Gel auf der Schleimhaut, das über 12 Stunden lang auf der Schleimhaut haftete und die Schleimhaut feucht hielt. Im Vergleich hielt das handelsübliche Präparat nur etwa eine halbe Stunde und mußte jede halbe Stunde aufgetragen werden, während das erfindungsgemäße Mittel nur zweimal am Tag aufgetragen wurde.

**Beispiel 30**

Ein Patient, 42 Jahre, litt unter trockener Nasenchleimhaut bei chronischer Rhinosinusitis. Ihm wurde Lösung A und Lösung B gemäß Beispiel 8 nacheinander in die Nase gesprüht. Das sich bildende Gel haftete mehrere Stunden lang auf der Nasenschleimhaut und hielt die Schleimhaut feucht.

**Beispiel 31**

Ein Patient, 45 Jahre, litt unter schwerer hämorrhagischer Refluxösophagitis (Stadium IV) und unter leichten Sickerblutungen. Über zwei verschiedene Sonden wurden nacheinander durch das Gastroskop zuerst die Lösung A und anschließend die Lösung B von Beispiel 14 appliziert. Auf der Schleimhaut bildete sich unmittelbar danach ein gelartiger Filmbelag, der die Läsionen abdeckte. Auch bei entstehender Peristaltik blieb dieser Film haften.

**Beispiel 32**

Mit zwei verschiedenen Sonden wurden durch das Gastroskop zuerst Lösung A und anschließend Lösung B nach Beispiel 18 auf ein Ulcus ventriculi appliziert. Auf der Schleimhaut bildete sich ein fester Gelfilm, der auch durch die Peristaltik des Magens nicht abgelöst wurde.

### Beispiel 33

Über zwei verschiedene Sonden wurden durch das Gastroskop nacheinander zuerst Lösung A und dann Lösung B von Beispiel 19 auf mehrere Ulcera ventriculi aufgebracht. Es bildete sich ein fester Gelfilm, der auf der Magenschleimhaut haften blieb.

### Beispiel 34

Über zwei Sonden wurden nacheinander zuerst Lösung A und gleich anschließend Lösung B von Beispiel 16 auf Sklerotisierungsulcera im distalen Ösophagus nach Varizenverödung aufgebracht. Der entstehende feste Gelfilm blieb mehrere Stunden auf der Schleimhaut haften.

### Beispiel 35

Über zwei verschiedene Sonden wurde zuerst Lösung A und anschließend Lösung B von Beispiel 23 auf die Schleimhaut des Colon eines Patienten aufgebracht, der unter Strahlencolitis mit profuser Sickerblutung litt. Der entstehende, sehr feste Gelfilm haftete mehrere Stunden auf der Schleimhaut und löste sich auch durch die Darmperistaltik nicht ab.

### Beispiel 36

Über zwei verschiedene Sonden wurden nacheinander Lösung A und anschließend Lösung B von Beispiel 22 auf den Teil der Colonschleimhaut aufgebracht, der durch Crohnläsionen geschädigt war. Der sich bildende, sehr feste Gelfilm blieb viele Stunden haften, so daß das in dem Gel als Trägermittel enthaltene Arzneimittel lokal an den geschädigten Stellen wirken konnte.


### Patentansprüche

### Patentansprüche für folgende Vertragsstaaten : BE, CH, LI, DE, FR, GB, IT, LU, NL, SE

1. Arzneim zur in-situ-Bildung mit guter Haftung auf der Schleimhaut, **dadurch gekennzeichnet,** daß es eine Losüng A mit einen Gehalt an einem Metallsalz, das zur Gelbildung mit einem Polysaccharid befähigt ist, und eine Lösung B mit einem Gehalt an einem zur Gelbildung befähigten Polysaccharid enthält.

2. Mittel, enthaltend als getrennt voneinander vorliegende Komponenten zur Bildungs eines Metallsalzes A, ein organisches Calciumsalz und B, als Polysaccharid Alginsäure, Polyguluronsäure, Polymannuronsäure, Propylenglykolalginsäure, Polygalacturonsäure, deren Salze oder Ester, Pektin oder deren Mischungen.

3. Mittel nach Anspruch 1 oder 2, **dadurch gekennzeichnet,** daß es zusätzlich Arzneistoffe, Desinfizienzia, Feuchthaltemittel und/oder Konservierungsmittel enthält.

4. Mittel nach einem der vorhergehenden Ansprüche, **dadurch gekennzeichnet,** daß die ein Metallsalz enthaltende Lösung in einer Konzentration von 0,01 bis 50 mmol Metall pro 100 ml und das Polysaccharid in einer Konzentration von 0,01 bis 12,5 Gew.-% vorhanden ist.

### Patentansprüche für folgenden Vertragsstaat : AT

1. Verfahren zur Herstellung eines Arzneimittels mit guter Haftung auf der Schleimhaut in situ, **dadurch gekennzeichnet,** daß man eine Lösung A mit einem Gehalt an einem Metallsalz, das zur Gelbildung mit einem Polysaccharid befähigt ist, auf die Schleimhaut aufbringt und anschlißend eine Lösung B mit einem Gehalt an einem zur Gelbildung befähigten Polysaccharid auf denselben Bereich der Schleimhaut aufbringt.

2. Verfahren nach Anspruch 1, **dadurch gekennzeichnet,** daß man zur Bildung eines Metallsalzes A) ein organisches Calciumsalz und B) als Polysaccharid Alginsäure, Polyguluronsäure, Polymannuronsäure, Propylenglykolalginsäure, Polygalacturonsäure, deren Salze oder Ester, Pektin oder deren Mischungen verwendet.

3. Verfahren nach Anspruch 1 oder 2, **dadurch gekennzeichnet,** daß Lösung A oder/und Lösung B zusätzlich Arzneistoffe, Desinfizienzia, Feuchthaltemittel und/oder Konservierungsmittel enthalten.

4. Verfahren nach einem der vorhergehenden Ansprüche, **dadurch gekennzeichnet,** daß die ein Metallsalz enthaltende Lösung in einer Konzentration von 0,01 bis 50 mmol Metall pro 100 ml und das Polysaccharid in einer Konzentration von 0,01 bis 12,5 Gew.-% verwendet wird.

Claims

Claims for the following Contracting States : BE, CH, LI, DE, FR, GB, IT, LU, NL, SE

1. Medicament for the in situ formation with good adhesion to the mucosa, characterised in that it contains a solution A with a content of a metal salt which is capable of gel formation with a polysaccharide and a solution B with a content of a polysaccharide capable of gel formation.

2. Agent containing as components, present separate from one another, for the formation of a metal salt, A an organic calcium salt and B, as polysaccharide, alginic acid, polyguluronic acid, polymannuronic acid, propyleneglycolalginic acid, polygalacturonic acid, their salts or esters, pectin or their mixture.

3. Agent according to claim 1 or 2, characterised in that it additionally contains medicaments, disinfectants, moisture-retaining agents and/or preservation agents.

4. Agent according to one of the preceding claims, characterised in that the solution containing a metal salt is present in a concentration of 0.01 to 50 mMole of metal per 100 ml. and the polysaccharide is present in a concentration of 0.01 to 12.5 wt.%.

Claims for the following Contracting State : AT

1. Process for the preparation in situ of a medicament with good adhesion to the mucosa, characterised in that one applies to the mucosa a Solution A with a content of a metal salt which is capable of gel formation with a polysaccharide and subsequently applies a solution B with a content of a polysaccharide capable of gel formation to the same region of the mucosa.

2. Process according to claim 1, characterised in that, for the formation of a metal salt, one uses A) an organic calcium salt and B), as polysaccharide, alginic acid, polyguluronic acid, polymannuronic acid, propyleneglycolalginic acid, polygalacturonic acid, their salts or esters, pectin or their mixtures.

3. Process according to claim 1 or 2, characterised in that solution A and/or solution B additionally contain medicaments, disinfectants, moisture-retaining agents and/or preservation agents.

4. Process according to one of the preceding claims, characterised in that the solution containing a metal salt is used in a concentration of 0.01 to 50 mMole of metal per 100 ml. and the polysaccharide in a concentration of 0.01 to 12.5 wt.%.

Revendications

Revendications pour les Etats contractants suivants : BE, CH, LI, DE, FR, GB, IT, LU, NL, SE

1. Médicament destiné à une formation in situ, présentant une bonne adhésivité aux muqueuses, caractérisé en ce qu'il contient une solution A contenant un sel métallique susceptible de former un gel avec un polysaccharide et une solution B contenant un polysaccharide susceptible de former un gel.

2. Agent contenant comme constituants présents en étant séparés l'un de l'autre pour former un sel métallique A) un sel organique du calcium et B) comme polysaccharide l'acide alginique, l'acide polyguluronique, l'acide polymannuronique, l'acide propylèneglycolalginique, l'acide polygalacturonique, leurs sels ou esters, la pectine ou leurs mélanges.

3. Agent selon la revendication 1 ou 2, caractérisé en ce qu'il contient en outre des médicaments, des désinfectants, des agents de maintien de l'humidité et/ou des conservateurs.

4. Agent selon l'une quelconque des revendications précédentes, caractérisé en ce que la solution qui contient un sel métallique est présente en une concentration de 0,01 à 50 mmoles de métal pour 100 ml et le polysaccharide est présent en une concentration de 0,01 à 12,5% en poids.

Revendications pour l'Etat contractant suivant : AT

1. Procédé d'obtention d'un médicament présentant une bonne adhésivité aux muqueuses in situ, caractérisé en ce que l'on applique sur les muqueuses une solution A contenant un sel métallique qui est susceptible de former un gel avec un polysaccharide, puis l'on applique sur le même domaine des muqueuses une solution B contenant un polysaccharide susceptible de former un gel.

2. Procédé selon la revendication 1, caractérisé en ce que l'on utilise pour former un sel métallique A) un sel organique du calcium et B) comme polysaccharide l'acide alginique, l'acide polyguluronique, l'acide polymannuronique, l'acide propylèneglycolalginique, l'acide polygalacturonique, leurs sels ou esters, la pectine ou leurs mélanges.

3. Procédé selon la revendication 1 ou 2, caractérisé en ce que la solution A et/ou la solution B contiennent en outre des médicaments, des désinfectants, des agents de maintien de l'humidité et/ou des conservateurs.

4. Procédé selon l'une quelconque des revendications précédentes, caractérisé en ce que la solution qui contient un sel métallique est utilisée en une concentration de 0,01 à 50 mmoles de métal pour 100 ml et le polysaccharide est utilisé en une concentration de 0,01 à 12,5% en poids.